# EUROPÄISCHE PATENTSCHRIFT

(11) **EP 3 870 051 B1**
(45) Veröffentlichungstag und Bekanntmachung des Hinweises auf die Patenterteilung: **06.11.2024**
(21) Anmeldenummer: 19769394.8
(22) Anmeldetag: 04.09.2019
(51) Int. Cl.: A61B 5/18, A61B 5/00

(54) **INSASSENÜBERWACHUNGSSYSTEM FÜR EIN FAHRZEUG**
OCCUPANT MONITORING SYSTEM FOR A VEHICLE
SYSTÈME DE SURVEILLANCE D'OCCUPANT D'UN VÉHICULE

(30) Priorität: 24.10.2018 DE 102018218215
(43) Veröffentlichungstag der Anmeldung: 01.09.2021
(73) Patentinhaber: Robert Bosch GmbH, 70442 Stuttgart (DE)
(72) Erfinder: TYROLT, Thomas, 70825 Korntal-Muenchingen (DE); WEISSENMAYER, Simon, 74223 Flein (DE)
(86) Internationale Anmeldenummer: PCT/EP2019/073581
(87) Internationale Veröffentlichungsnummer: WO 2020/083549

(56) Entgegenhaltungen:
- DE-A1- 102011 016 772
- DE-A1- 102011 110 486
- DE-A1- 102013 223 439
- DE-A1- 102014 211 501
- DE-A1- 102016 011 655
- DE-T5- 112016 002 832

## Beschreibung

Die Erfindung geht aus von einem Insassenüberwachungssystem für ein Fahrzeug nach der Gattung des unabhängigen Patentanspruchs 1.

Um Verletzungen durch den Airbag bei sogenannten Out-of-Position-Situationen zu vermeiden, verwenden aus dem Stand der Technik bekannte Insassenschutzsysteme Insassenüberwachungssysteme mit mindestens einer Innenraumsensorik, um Position und Körperhaltung von Insassen zu ermitteln.

Dadurch kann vor dem Auslösen des Airbags erkannt werden, ob sich ein Insasse im Entfaltungsbereich des Airbags befindet. In der Regel umfasst eine solche Innenraumsensorik optische Sensoren bzw. Kameras um die aktuelle Position und Körperhaltung der Insassen zu erfassen. Insbesondere bei Fahrzeugen mit autonomen Fahrfunktionen kann dem Fahrer erlaubt werden, während der automatisierten Fahrt viele verschiedene Positionen einzunehmen und Tätigkeiten auszuführen. So kann der Fahrer beispielsweise eine Virtual-Reality-Brille benutzen, elektrische Geräte wie Smartphone, Tablet, Laptop usw. bedienen, telefonieren ohne Freisprecheinrichtung (mit Smartphone am Ohr), lesen (Buch, Brief, Zeitung usw.), malen, essen und/oder trinken. Zudem kann die Lehne in Richtung Schlafposition verstellt sein oder der Sitz weit nach hinten gefahren sein oder Füße auf den Sitz angezogen sein.

Aus der DE 10 2014 211 501 A1 ist ein Insassenüberwachungssystem nach dem Oberbegriff des Anspruchs 1 bekannt.

Aus der DE 10 2008 002 232 A1 sind ein adaptives Verfahren und eine zur Durchführung dieses Verfahrens geeignete Vorrichtung zur Bestimmung von Entfernung und/oder Geschwindigkeit eines Objekts relativ zu einem Fahrzeug bekannt. Dabei werden Ultraschallpulse ausgesandt, von dem Objekt reflektiert und als Echo wieder empfangen. Das Aussenden eines zweite Ultraschallpulses wird durch den Empfang des Echos eines zuvor ausgesandten ersten Ultraschallpulses getriggert, wodurch in dynamischen Situationen wertvolle Zeit gewonnen wird.

### Offenbarung der Erfindung

Das Insassenüberwachungssystem für ein Fahrzeug mit den Merkmalen des unabhängigen Patentanspruchs 1 hat den Vorteil, dass durch Ultraschallsensoren und/oder Mikrofone einer Innenraumsensorik Atemgeräusche der Insassen ermittelt und zur Bestimmung des körperlichen Zustands der einzelnen Insassen ausgewertet werden können. Zudem können über die Ultraschallsensoren und/oder die Mikrofone Position und Tätigkeit der einzelnen Insassen bestimmt werden.

Turbulente Windgeräusche, wie sie beim Atmen entstehen können, erzeugen Ultraschallsignale in einem Frequenzbereich, welcher von herkömmlichen Ultraschallsensoren und/oder Mikrofonen erfasst werden kann. Hierbei entstehen beim Atmen viele charakteristische Resonanzfrequenzen ähnlich wie beim Sprechen.

Ausführungsformen der vorliegenden Erfindung stellen ein Insassenüberwachungssystem für ein Fahrzeug zur Verfügung, welches mindestens eine autonome Fahrfunktion aufweist. Das Insassenüberwachungssystem für ein Fahrzeug umfasst mindestens eine Sensorik zur Erfassung eines aktuellen Fahrzustands des Fahrzeug, eine Innenraumsensorik zur Erfassung eines Innenraumzustands und mindestens eine Auswerte- und Steuereinheit. Hierbei umfasst die Innenraumsensorik mehrere verteilt im Fahrzeuginnenraum angeordnete Ultraschallsensoren und/oder Mikrofone, wobei die Auswerte- und Steuereinheit Rohsignale der Ultraschallsensoren und/oder der Mikrofone empfängt und weiterverarbeitet. Die Auswerte- und Steuereinheit ermittelt basierend auf den Rohsignalen der Ultraschallsensoren und/oder der Mikrofone Atemgeräusche der Insassen, welche charakteristische Resonanzfrequenzen aufweisen.

Unter der Auswerte- und Steuereinheit kann vorliegend ein elektrisches Gerät, wie beispielsweise ein Steuergerät, insbesondere ein Airbagsteuergerät, verstanden werden, welches erfasste Sensorsignale verarbeitet bzw. auswertet. Die Auswerte- und Steuereinheit kann mindestens eine Schnittstelle aufweisen, die hard- und/oder softwaremäßig ausgebildet sein kann. Bei einer hardwaremäßigen Ausbildung können die Schnittstellen beispielsweise Teil eines sogenannten System-ASICs sein, der verschiedenste Funktionen der Auswerte- und Steuereinheit beinhaltet. Es ist jedoch auch möglich, dass die Schnittstellen eigene, integrierte Schaltkreise sind oder zumindest teilweise aus diskreten Bauelementen bestehen. Bei einer softwaremäßigen Ausbildung können die Schnittstellen Softwaremodule sein, die beispielsweise auf einem Mikrocontroller neben anderen Softwaremodulen vorhanden sind. Von Vorteil ist auch ein Computerprogrammprodukt mit Programmcode, der auf einem maschinenlesbaren Träger wie einem Halbleiterspeicher, einem Festplattenspeicher oder einem optischen Speicher gespeichert ist und zur Durchführung der Auswertung verwendet wird, wenn das Programm von der Auswerte- und Steuereinheit ausgeführt wird.

Unter der Sensorik zur Erfassung eines aktuellen Fahrzustands des Fahrzeug werden nachfolgend Sensorvorrichtungen verstanden, welche beispielsweise einen automatischen Fahrbetrieb des Fahrzeugs unterstützen oder eine Umgebung des Fahrzeugs zur Crasherkennung erfassen.

Im Gegensatz zu Innenraumkamerasystemen sind die Ultraschallsensoren und/oder Mikrofone unempfindlich gegenüber Schmutz und Staub und können nicht von anderen Fahrzeugen geblendet werden. Auch kreative Muster von Sitzbezügen oder z.B. bedruckte Kleidung haben keinen Einfluss auf die Atmungserkennung. Des Weiteren sind Ultraschallsensoren äußerst kostengünstig. In vorteilhafter Weise kann eine automatisierte Fahrt besser abgesichert werden, da aufgrund einer aus den Atemgeräuschen ermittelten Atemfrequenz auf einen Stresspegel des Fahrers und damit auf den körperlichen Gesundheitszustand des Fahrers geschlossen werden kann. Dadurch kann die Auswerte- und Steuereinheit entscheiden, ob dem Fahrer die Kontrolle übergeben werden kann oder ob das Fahrzeug sicher abgestellt wird. Des Weiteren kann durch den Einsatz der Ultraschallsensoren und/oder der Mikrofone in Verbindung mit der Atemgeräuschermittlung auf Sitzbelegungssensoren verzichtet werden. Im Gegensatz zum Einsatz von Kameras fühlen sich die Insassen durch die Verwendung von Ultraschallsensoren nicht in ihrer Privatsphäre gestört.

Durch die in den abhängigen Ansprüchen aufgeführten Maßnahmen und Weiterbildungen sind vorteilhafte Verbesserungen des im unabhängigen Patentanspruch 1 angegebenen Insassenüberwachungssystems für ein Fahrzeug möglich.

Besonders vorteilhaft ist, dass erste Ultraschallsensoren und/oder erste Mikrofone zum Erfassen der Atemgeräusche der Insassen an luftverwirbelungsarmen Bereichen im Innenraum angeordnet werden können. Zudem können zweite Ultraschallsensoren und/oder zweite Mikrofone zur Erfassung von Störgeräuschen vorgesehen werden, wobei die Auswerte- und Steuereinheit die Rohsignale der zweiten Ultraschallsensoren und/oder der zweiten Mikrofone zur Kompensation von Störgeräuschen bei der Auswertung der Atemgeräusche verwenden kann.

In weiterer vorteilhafter Ausgestaltung des Insassenüberwachungssystems kann die Auswerte- und Steuereinheit die Rohsignale der Ultraschallsensoren und/oder der Mikrofone mit sich selbst falten und/oder per Autokorrelation verrechnen, um die charakteristische Resonanzfrequenzen und Atemfrequenzen der einzelnen Insassen aus den Atemgeräuschen zu extrahieren. Des Weiteren kann die Auswerte- und Steuereinheit durch Auswerten der charakteristischen Resonanzfrequenzen und Atemfrequenzen eine Insassenanzahl und einen körperlichen Gesundheitszustand der einzelnen Insassen bestimmen. Auch der Stresslevel der Insassen, insbesondere des Fahrers kann anhand der Atemgeräusche erkannt und ausgewertet werden. Dabei lassen nicht nur Atemfrequenz und Volumenstrom bzw. die gemessene Rauschintensität auf den Stresslevel schließen, sondern auch die charakteristischen Resonanzfrequenzen. Die unterschiedlichen Resonanzfrequenzen entstehen, weil die Schleimhäute in der Nase bei Anspannung (z.B. durch Adrenalin) abschwellen und bei Entspannung anschwellen und dadurch Einfluss auf die Resonanzvolumen nehmen können. Die Auswerte- und Steuereinheit kann Rohdaten von den verbauten Ultraschallsensoren im Innenraum empfangen und auswerten, um die Atemgeräusche zu erkennen und zu lokalisieren. Auch Windgeräusche von offenen Fenstern und Geräusche beim Befahren von nassen Straßen und Pfützen, sowie Ultraschallgeräusche aus dem Motorraum werden von den Ultraschallsensoren und/oder den Mikrofonen als Rauschen erfasst und überlagern die Atemgeräusche. In vorteilhafter Weise kann die Auswerte- und Steuereinheit daher auch Rohdaten von äußeren Ultraschallsensoren und/oder Mikrofonen und/oder von Ultraschallsensoren und/oder Mikrofonen aus dem Motorraum für eine Datenfusion verwenden, um die Atemgeräusche noch besser erfassen und extrahieren zu können. Die Datenfusion mit Ultraschallsensoren und/oder Mikrofonen aus dem Motorraum und der Umgebung verbessert natürlich auch die Objekterkennung.

In weiterer vorteilhafter Ausgestaltung des Insassenüberwachungssystems kann die Auswerte- und Steuereinheit nach einem Unfall den körperlichen Gesundheitszustand der einzelnen Insassen zusammen mit einem Notruf an eine Notrufzentrale übermitteln.

In weiterer vorteilhafter Ausgestaltung des Insassenüberwachungssystems kann die Auswerte- und Steuereinheit durch Fusion und Auswertung der Rohsignale Position und Körperhaltung der einzelnen Insassen bestimmt. Des Weiteren kann die Auswerte- und Steuereinheit während einer automatisierten Fahrt basierend auf der Körperhaltung und dem körperlichen Gesundheitszustand eines Fahrers entscheiden, ob dem Fahrer die Kontrolle über das Fahrzeug wieder übergeben werden kann, oder ob das Fahrzeug automatisiert sicher abgestellt wird.

In weiterer vorteilhafter Ausgestaltung des Insassenüberwachungssystems kann die Auswerte- und Steuereinheit durch Echoortung Positionen von Gesichtern der einzelnen Insassen berechnen. Basierend auf der Position und Körperhaltung der einzelnen Insassen kann ein übergeordnetes Insassenschutzsystem während eines Unfalls entscheiden, welche Schutzmittel zum Schutz der Insassen aktiviert werden. Dadurch können beispielsweise Airbags bei einem Unfall nur dann entfalten werden, wenn aufgrund des ermittelten körperlichen Gesundheitszustands, der Körperhaltung und der Tätigkeit der Insassen durch den sich entfaltenden Airbag keine Gefährdung der Insassen hervorgerufen wird.

In weiterer vorteilhafter Ausgestaltung des Insassenüberwachungssystem s kann die Innenraumsensorik mindestens einen weiteren Innenraumsensor umfassen, dessen Signale die Auswerte- und Steuereinheit zur Plausibilisierung und/oder zur Optimierung des Auswerteergebnisses der Rohsignale der Ultraschallsensoren verwenden kann. Der mindestens eine weitere Innenraumsensor kann beispielsweise als Sitzpositionssensor oder als optischer Sensor oder Lidarsystem ausgeführt werden. Da die Sitzverstellung einen großen Einfluss auf die Position der Personen und deren Körperhaltung hat, können die Informationen von elektrisch verstellbaren Sitzen zur Plausibilisierung oder Optimierung der Objekterkennung herangezogen werden. Der Abstand zwischen Gesicht und den Ultraschallsensoren bzw. den Mikrofonen hat großen Einfluss auf die Dämpfung des gemessenen Rauschpegels. Zusätzlich kann mit Hilfe der Echoortung die Position der Gesichter der Insassen erfasst werden, um damit die gemessene Intensität des Rauschpegels zu kompensieren.

Durch Fusion der Sensorsignale kann die Auswerte- und Steuereinheit bei Ausführungsformen der Erfindung erkennen, auf welchen Plätzen Personen im Fahrzeug sitzen, wie groß diese Personen sind und welche Körperhaltung die Personen einnehmen. Daraufhin kann die Auswerte- und Steuereinheit bei einem Unfall entscheiden, welche Schutzmittel ausgelöst werden. Bei der automatisierten Fahrt kann die Auswerte- und Steuereinheit aufgrund der erkannten Körperhaltung bzw. einem erkannten Fahrerzustand entscheiden, ob dem Fahrer vor dem Beenden der automatisierten Fahrt die Kontrolle zurückgeben werden kann oder ob das Fahrzeug sicher abgestellt wird. Kann die Auswerte- und Steuereinheit dem Fahrer die Kontrolle über das Fahrzeug nicht zurückgegeben werden, weil beispielsweise die Hände des Fahrers nicht am Lenkrad sind, oder der Fahrer einen hohen Stresspegel aufweist oder die Füße des Fahrers nicht im Fußraum sind, dann kann die Auswerte- und Steuereinheit die Fahrt durch einen Halt am Straßenrand oder auf dem Seitenstreifen beenden. Zuvor kann die Auswerte- und Steuereinheit beispielsweise mindestens eine vorzugsweise mehrere optische und/oder akustische Warnsignale ausgeben.

Ein Ausführungsbeispiel der Erfindung ist in der Zeichnung dargestellt und wird in der nachfolgenden Beschreibung näher erläutert. In der Zeichnung bezeichnen gleiche Bezugszeichen Komponenten bzw. Elemente, die gleiche bzw. analoge Funktionen ausführen.

### Kurze Beschreibung der Zeichnung

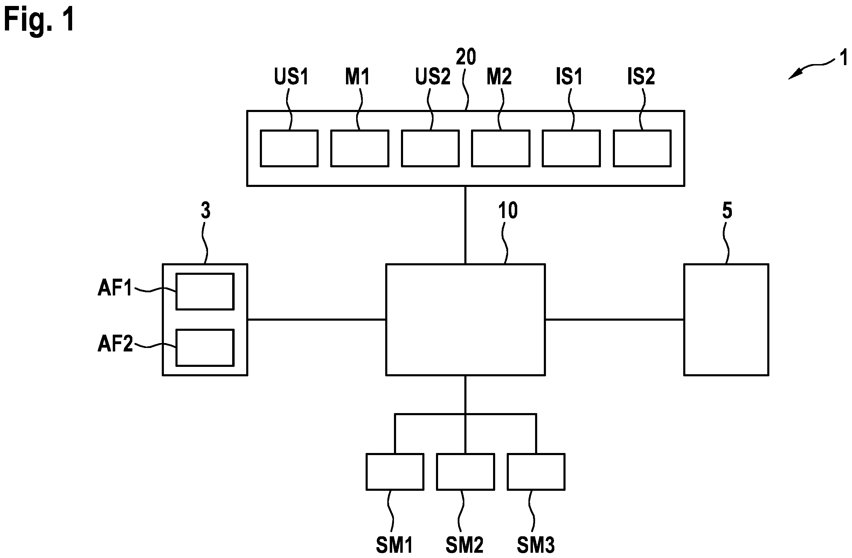

Fig. 1 zeigt ein schematisches Blockdiagramm eines Ausführungsbeispiels eines erfindungsgemäßen Insassenüberwachungssystems für ein Fahrzeug.

### Ausführungsformen der Erfindung

Wie aus Fig. 1 ersichtlich ist, umfasst das dargestellte Ausführungsbeispiel eines erfindungsgemäßen Insassenüberwachungssystems 1 für ein Fahrzeug mindestens eine Sensorik 5 zur Erfassung eines aktuellen Fahrzustands des Fahrzeugs, eine Innenraumsensorik 20 zur Erfassung eines Innenraumzustands und mindestens eine Auswerte- und Steuereinheit 10. Hierbei umfasst die Innenraumsensorik 20 mehrere verteilt im Fahrzeuginnenraum angeordnete Ultraschallsensoren US1, US2 und/oder Mikrofone M1, M2, wobei die Auswerte- und Steuereinheit 10 Rohsignale der Ultraschallsensoren US1, US2 und/oder der Mikrofone M1, M2 empfängt und weiterverarbeitet. Die Auswerte- und Steuereinheit 10 ermittelt basierend auf den Rohsignalen der Ultraschallsensoren US1, US2 und/oder der Mikrofone M1, M2 Atemgeräusche der Insassen, welche charakteristische Resonanzfrequenzen aufweisen. Das Fahrzeug 1 umfasst im dargestellten Ausführungsbeispiel ein Fahrerassistenzsystem 3, welches mindestens eine autonome Fahrfunktion AF1, AF2 zur Durchführung eines vollautomatischen oder zumindest eines teilautomatischen Fahrbetriebs aufweist, und ein übergeordnetes nicht näher bezeichnete Insassenschutzsystem, welchem mindestens ein Insassenschutzmittel SM1, SM2, SM3 zugeordnet ist.

Die Innenraumsensorik 20 umfasst im dargestellten Ausführungsbeispiel erste Ultraschallsensoren US1 und erste Mikrofone M1 zum Erfassen der Atemgeräusche der Insassen, welche an luftverwirbelungsarmen Bereichen im Innenraum angeordnet sind, und zweite Ultraschallsensoren US2 und zweite Mikrofone M2 zur Erfassung von Störgeräuschen. Hierbei verwendet die Auswerte- und Steuereinheit 10 die Rohsignale der zweiten Ultraschallsensoren US2 und der zweiten Mikrofone M2 zur Kompensation von Störgeräuschen bei der Auswertung der Atemgeräusche. Bei einem nicht dargestellten Ausführungsbeispiel umfasst die Innenraumsensorik 20 nur Ultraschallsensoren US1, US2. Bei einem weiteren nicht dargestellten Ausführungsbeispiel umfasst die Innenraumsensorik 20 nur Mikrofone M1, M2. Bei einem weiteren nicht dargestellten Ausführungsbeispiel umfasst die Innenraumsensorik 20 Ultraschallsensoren U1 zur Erfassung der Atemgeräusche und Mikrofone M2 zur Erfassung der Störgeräusche. Bei noch einem weiteren nicht dargestellten Ausführungsbeispiel umfasst die Innenraumsensorik 20 Mikrofone M1 zur Erfassung der Atemgeräusche und Ultraschallsensoren U1 zur Erfassung der Störgeräusche

Als Montageorte für die Ultraschallsensoren U1, U2 und/oder die Mikrofone M1, M2 eignen sich vor allem Plätze, welche ausreichend voluminös sind, um das Gehäuse der Ultraschallsensoren U1, U2 und/oder der Mikrofone M1, M2 aufnehmen können, so dass die Sensorfläche plan auf der Oberfläche des Innenraums aufliegen kann und die restlichen Komponenten bis auf die Sensorfläche unsichtbar hinter der Verkleidung verschwinden. Zudem werden die Ultraschallsensoren U1, U2 und/oder die Mikrofone M1, M2 so angebracht, dass bewegliche Teile des Innenraums (Sitze, Lenkrad, etc.) und Körperteile von Insassen möglichst weit entfernt sind. Da die ersten Ultraschallsensoren U1 und/oder die ersten Mikrofone M1 bei der Erkennung von Atemgeräuschen von anderen Luftverwirbelungen gestört werden können, werden die ersten Ultraschallsensoren U1 und/oder die ersten Mikrofone M1 außerdem entfernt von Lüftungsschlitzen und Fensteröffnungen angebracht. Zudem sind die Sensorflächen bei planer Auflage mit der Innenverkleidung möglichst gut in Richtung der Körperteile der Insassen ausgerichtet. Vor diesem Hintergrund eignen sich vor allem die Einbauorte im Bereich eines Innenspiegels und im Bereich einer Innenraumbeleuchtung. Auch A-Säule, B-Säule, und C-Säule können als Einbauort für die zweiten Ultraschallsensoren U2 und/oder die zweiten Mikrofone M2 verwendet werden, um durch offene Fenstern verursachte Luftverwirbelungen als Störgeräusche zu erfassen. Auch eine Anbringung der ersten Ultraschallsensoren U1 und/oder der ersten Mikrofone M1 im Armaturenbrett, möglichst entfernt von den Lüftungsschlitzen ist günstig. Hier ist es auch denkbar, die Ultraschallsensoren U1, U2 gegen eine Windschutzscheibe zu richten, so dass diese den Schall auf Fahrer und Beifahrer reflektiert, um damit die Entfernung zu den Insassen und zu Luftverwirbelungen zu erhöhen.

Für die Objekterkennung innerhalb des Fahrzeugs können generell die gleichen Ultraschallsensoren U1, U2 verwendet werden, wie außerhalb des Fahrzeugs. Allerdings ist im Innenraum eine wesentlich geringere Reichweite der Ultraschallsensoren U1, U2 notwendig, weshalb Leistungseinbußen möglich sind und daher die Ultraschallsensoren U1, U2 auch unsichtbar hinter der Verkleidung angebracht werden können. Generell sind wegen der kürzeren Distanzen im Innenraum kürzere Echosignale zu bevorzugen, da die Ultraschallsensoren U1, U2 eine gewisse Ausschwingzeit benötigen. Daher können Objekte mit zu kurzen Abständen zu den Ultraschallsensoren U1, U2 nicht durch einen einzigen Ultraschallsensor U1, U2 erkannt werden. Es ist allerdings möglich verstärkt mit Kreuzechos Objekte zu erkennen, da hier das Echo des einen Ultraschallsensors U1, U2 von einem anderen Ultraschallsensor U1, U2 empfangen wird, und die Ausschwingzeit somit keine Rolle spielt.

Im dargestellten Ausführungsbeispiel faltet die Auswerte- und Steuereinheit 10 die Rohsignale der Ultraschallsensoren US1, US2 und/oder der Mikrofone M1, M2 mit sich selbst, um die charakteristische Resonanzfrequenzen und Atemfrequenzen der einzelnen Insassen aus den Atemgeräuschen zu extrahieren. Zusätzlich oder alternativ kann die Auswerte- und Steuereinheit 10 die Rohsignale der Ultraschallsensoren US1, US2 und/oder Mikrofone M1, M2 per Autokorrelation verrechnen. Auf diese Art können Resonanzfrequenzen im Ultraschallbereich besonders gut detektiert werden. Durch Auswerten der charakteristischen Resonanzfrequenzen und Atemfrequenzen bestimmt die Auswerte- und Steuereinheit 10 im dargestellten Ausführungsbeispiel eine Insassenanzahl und einen körperlichen Gesundheitszustand der einzelnen Insassen. Zudem können per Mustererkennung der Resonanzfrequenzen die Insassen ähnlich wie mit Hilfe eines Fingerabdrucks identifiziert werden. Daher ist es auch möglich die Zugangsberechtigung zum Fahrzeug anhand der Atemgeräusche zu überprüfen. Auch der Stresslevel des Fahrers kann anhand der Atemgeräusche erkannt werden. Dabei lassen nicht nur Atemfrequenz und Volumenstrom bzw. die gemessene Rauschintensität auf den Stresslevel schließen, sondern auch die charakteristischen Resonanzfrequenzen.

Im dargestellten Ausführungsbeispiel übermittelt die Auswerte- und Steuereinheit 10 nach einem Unfall den körperlichen Gesundheitszustand der einzelnen Insassen zusammen mit einem Notruf an eine Notrufzentrale. Daraus kann die Rettungsleitstelle einen ersten Eindruck über den möglichen Gesundheitszustand bzw. die körperliche Verfassung der einzelnen Insassen bekommen.

Zudem bestimmt die Auswerte- und Steuereinheit durch Fusion und Auswertung der Rohsignale Position und Körperhaltung der einzelnen Insassen. Basierend auf der Körperhaltung und dem körperlichen Gesundheitszustand des Fahrers entscheidet die Auswerte- und Steuereinheit 10 während einer automatisierten Fahrt, ob dem Fahrer die Kontrolle über das Fahrzeug wieder übergeben werden kann, oder ob das Fahrzeug automatisiert sicher abgestellt wird. Zur Verbesserung der Bestimmung der Position und/oder der Körperhaltung der Insassen berechnet die Auswerte- und Steuereinheit 10 im dargestellten Ausführungsbeispiel durch Echoortung Positionen von Gesichtern der einzelnen Insassen. Basierend auf der Position und Körperhaltung der einzelnen Insassen entscheidet das übergeordnete Insassenschutzsystem 1 während eines Unfalls, welche Schutzmittel SM1, SM2, SM3 wie beispielsweise Gurtstraffer, Airbags usw. zum Schutz der Insassen aktiviert werden.

Im dargestellten Ausführungsbeispiel umfasst die Innenraumsensorik 20 weitere Innenraumsensoren IS1, IS2, deren Signale die Auswerte- und Steuereinheit 10 zur Plausibilisierung und/oder zur Optimierung des Auswerteergebnisses der Rohsignale der Ultraschallsensoren US1, US2 und/oder der Mikrofone M1, M2 verwendet werden. Da auch die Sitzverstellung einen großen Einfluss auf die Position der Personen und deren Körperhaltung hat, liefern die weiteren Innenraumsensoren IS1, IS2 im dargestellten Ausführungsbeispiel Informationen von elektrisch verstellbaren Sitzen zur Plausibilisierung oder Optimierung der Objekterkennung. Der Abstand zwischen Gesicht und den Ultraschallsensoren U1, U2 und/oder den Mikrofonen M1, M2 hat großen Einfluss auf die Dämpfung des gemessenen Rauschpegels. Daher werden die erfassten Positionen der Gesichter der Insassen zusätzlich eingesetzt, um die gemessene Intensität des Rauschpegels zu kompensieren. Zusätzlich oder alternativ können optische Sensoren wie Kameras oder ein Lidarsystem als weitere Innenraumsensoren IS1, IS2 verwendet werden, um die Position und Körperhaltung der einzelnen Insassen zu ermitteln. Allerdings ist die Entwicklung von Algorithmen zur robusten Objekterkennung durch die große Störanfälligkeit der Kameras sehr teuer. Der Einsatz von Lidar ist auch sehr aufwändig und daher teuer. Optische Verfahren haben generell den Nachteil, dass beim Auslösen der Airbags Staub entsteht, welcher sich auf Linsen legen kann und die Sicht und die Objekterkennung beeinträchtigen kann. Außerdem müssen die Linsen regelmäßig von Staub und Schmutz gereinigt werden, um die Funktionsfähigkeit zu gewährleisten. Des Weiteren haben optische Systeme Probleme bei schwarzer Kleidung.

## Patentansprüche

1. Insassenüberwachungssystem (1) für ein Fahrzeug, welches mindestens eine autonome Fahrfunktion (AF1, AF2) aufweist, mit mindestens einer Sensorik (5) zur Erfassung eines aktuellen Fahrzustands des Fahrzeug, einer Innenraumsensorik (20) zur Erfassung eines Innenraumzustands und mindestens einer Auswerte- und Steuereinheit (10), **dadurch gekennzeichnet, dass** die Innenraumsensorik (20) mehrere verteilt im Fahrzeuginnenraum angeordnete Ultraschallsensoren (US1, US2) und/oder Mikrofone (M1, M2) umfasst, wobei die Auswerte- und Steuereinheit (10) Rohsignale der Ultraschallsensoren (US1, US2) und/oder der Mikrofone (M1, M2) empfängt und weiterverarbeitet, wobei die Auswerte- und Steuereinheit (10) basierend auf den Rohsignalen der Ultraschallsensoren (US1, US2) und/oder der Mikrofone (M1, M2) Atemgeräusche der Insassen ermittelt, welche charakteristische Resonanzfrequenzen aufweisen und dass die Auswerte- und Steuereinheit (10) die Rohsignale der Ultraschallsensoren (US1, US2) und/oder der Mikrofone (M1, M2) mit sich selbst faltet und/oder per Autokorrelation verrechnet, um die charakteristische Resonanzfrequenzen und Atemfrequenzen der einzelnen Insassen aus den Atemgeräuschen zu extrahieren, wobei die Auswerte- und Steuereinheit (10) durch Auswerten der charakteristischen Resonanzfrequenzen und Atemfrequenzen eine Insassenanzahl und einen körperlichen Gesundheitszustand der einzelnen Insassen bestimmt.

2. Insassenüberwachungssystem (1) nach Anspruch 1, **dadurch gekennzeichnet, dass** erste Ultraschallsensoren (US1) und/oder erste Mikrofone (M1) zum Erfassen der Atemgeräusche der Insassen an luftverwirbelungsarmen Bereichen im Innenraum angeordnet sind, und zweite Ultraschallsensoren (US2) und/oder zweite Mikrofone (M2) zur Erfassung von Störgeräuschen vorgesehen sind, wobei die Auswerte- und Steuereinheit (10) die Rohsignale der zweiten Ultraschallsensoren (US2) und/oder der zweiten Mikrofone (M2) zur Kompensation von Störgeräuschen bei der Auswertung der Atemgeräusche verwendet.

3. Insassenüberwachungssystem (1) nach Anspruch 1 oder 2, **dadurch gekennzeichnet, dass** die Auswerte- und Steuereinheit (10) nach einem Unfall den körperlichen Gesundheitszustand der einzelnen Insassen zusammen mit einem Notruf an eine Notrufzentrale übermittelt.

4. Insassenüberwachungssystem (1) nach einem der Ansprüche 1 bis 3, **dadurch gekennzeichnet, dass** die Auswerte- und Steuereinheit (10) durch Fusion und Auswertung der Rohsignale Position und Körperhaltung der einzelnen Insassen bestimmt.

5. Insassenüberwachungssystem (1) nach Anspruch 1 oder 2 und 4, **dadurch gekennzeichnet, dass** die Auswerte- und Steuereinheit (10) während einer automatisierten Fahrt basierend auf der Körperhaltung und dem körperlichen Gesundheitszustand eines Fahrers entscheidet, ob dem Fahrer die Kontrolle über das Fahrzeug wieder übergeben wird, oder ob das Fahrzeug automatisiert sicher abgestellt wird.

6. Insassenüberwachungssystem (1) nach Anspruch 4 oder 5, **dadurch gekennzeichnet, dass** die Auswerte- und Steuereinheit (10) durch Echoortung Positionen von Gesichtern der einzelnen Insassen berechnet.

7. Insassenüberwachungssystem (1) nach Anspruch 1 bis 6, **dadurch gekennzeichnet, dass** die Innenraumsensorik (20) mindestens einen weiteren Innenraumsensor (IS1, IS2) umfasst, dessen Signale die Auswerte- und Steuereinheit (10) zur Plausibilisierung und/oder zur Optimierung des Auswerteergebnisses der Rohsignale der Ultraschallsensoren (US1, US2) und/oder der Mikrofone (M1, M2) verwendet.

8. Insassenüberwachungssystem (1) nach Anspruch 7, **dadurch gekennzeichnet, dass** der mindestens eine weitere Innenraumsensor (IS1, IS2) als Sitzpositionssensor oder als optischer Sensor oder Lidarsystem ausgeführt ist.

9. Insassenschutzsystem umfassend ein Insassenüberwachungssystem (1) nach einem der Ansprüche 4 bis 8, wobei das Insassenschutzsystem während eines Unfalls basierend auf der Position und Körperhaltung der einzelnen Insassen entscheidet, welche Schutzmittel (SM1, SM2, SM3) zum Schutz der Insassen aktiviert werden.

## Claims

1. Occupant monitoring system (1) for a vehicle that has at least one autonomous driving function (AF1, AF2), having at least one sensor system (5) for detecting a current driving state of the vehicle, an interior sensor system (20) for detecting an interior state and at least one evaluation and control unit (10), **characterized in that** the interior sensor system (20) comprises multiple ultrasonic sensors (US1, US2) and/or microphones (M1, M2) arranged in the vehicle interior in a distributed manner, wherein the evaluation and control unit (10) receives raw signals of the ultrasonic sensors (US1, US2) and/or of the microphones (M1, M2) and processes said signals further, wherein the evaluation and control unit (10) takes the raw signals of the ultrasonic sensors (US1, US2) and/or of the microphones (M1, M2) as a basis for determining breathing sounds from the occupants, which have characteristic resonant frequencies, and wherein the evaluation and control unit (10) convolutes the raw signals of the ultrasonic sensors (US1, US2) and/or of the microphones (M1, M2) with itself and/or accounts for said signals by autocorrelation in order to extract the characteristic resonant frequencies and breathing frequencies of the individual occupants from the breathing sounds, the evaluation and control unit (10) evaluating the characteristic resonant frequencies and breathing frequencies in order to determine a number of occupants and a physical state of health of the individual occupants.

2. Occupant monitoring system (1) according to Claim 1, **characterized in that** first ultrasonic sensors (US1) and/or first microphones (M1) for capturing the breathing sounds of the occupants are arranged in areas with low turbulence in the interior, and there is provision for second ultrasonic sensors (US2) and/or second microphones (M2) for capturing noise, wherein the evaluation and control unit (10) uses the raw signals of the second ultrasonic sensors (US2) and/or of the second microphones (M2) to compensate for noise when evaluating the breathing sounds.

3. Occupant monitoring system (1) according to Claim 1 or 2, **characterized in that** the evaluation and control unit (10) relays the physical state of health of the individual occupants to an emergency centre together with an emergency call following an accident.

4. Occupant monitoring system (1) according to one of Claims 1 to 3, **characterized in that** the evaluation and control unit (10) determines the position and body posture of the individual occupants by fusing and evaluating the raw signals.

5. Occupant monitoring system (1) according to Claims 1 or 2 and 4, **characterized in that** during an automated journey the evaluation and control unit (10) takes the body posture and the physical state of health of a driver as a basis for deciding whether control of the vehicle is transferred back to the driver or whether the vehicle is safely parked in an automated manner.

6. Occupant monitoring system (1) according to Claim 4 or 5, **characterized in that** the evaluation and control unit (10) uses echolocation to compute positions of faces of the individual occupants.

7. Occupant monitoring system (1) according to Claims 1 to 6, **characterized in that** the interior sensor system (20) comprises at least one further interior sensor (IS1, IS2), the signals of which are used by the evaluation and control unit (10) to check the plausibility of and/or optimize the evaluation result pertaining to the raw signals of the ultrasonic sensors (US1, US2) and/or of the microphones (M1, M2).

8. Occupant monitoring system (1) according to Claim 7, **characterized in that** the at least one further interior sensor (IS1, IS2) is a seat position sensor or an optical sensor or a lidar system.

9. Occupant protection system comprising an occupant monitoring system (1) according to one of Claims 4 to 8, wherein during an accident the occupant protection system takes the position and body posture of the individual occupants as a basis for deciding which protective means (SM1, SM2, SM3) are activated to protect the occupants.

## Revendications

1. Système de surveillance d'occupant (1) pour un véhicule qui présente au moins une fonction de conduite autonome (AF1, AF2), comprenant au moins un système de capteurs (5) pour détecter un état de conduite actuel du véhicule, un système de capteurs d'habitacle (20) pour détecter un état d'habitacle et au moins une unité d'évaluation et de commande (10), **caractérisé en ce que** le système de capteurs d'habitacle (20) comprend plusieurs capteurs à ultrasons (US1, US2) et/ou microphones (M1, M2) répartis dans l'habitacle du véhicule, dans lequel l'unité d'évaluation et de commande (10) reçoit et traite des signaux buts des capteurs à ultrasons (US1, US2) et/ou des microphones (M1, M2), dans lequel l'unité d'évaluation et de commande (10) établit sur la base des signaux bruts des capteurs à ultrasons (US1, US2) et/ou des microphones (M1, M2) des bruits de respiration des occupants qui présentent des fréquences de résonance caractéristiques, et **en ce que** l'unité d'évaluation et de commande (10) convolute les signaux bruts des capteurs à ultrasons (US1, US2) et/ou des microphones (M1, M2) avec eux-mêmes et/ou les calcule par autocorrélation afin d'extraire des bruits de respiration les fréquences de résonance et les fréquences respiratoires caractéristiques des différents occupants, dans lequel l'unité d'évaluation et de commande (10) détermine par évaluation des fréquences de résonance et fréquences respiratoires caractéristiques un nombre d'occupants et un état de santé corporel des différents occupants.

2. Système de surveillance d'occupant (1) selon la revendication 1, **caractérisé en ce que** des premiers capteurs à ultrasons (US1) et/ou des premiers microphones (M1) pour détecter les bruits de respiration des occupants sont disposés dans des zones avec peu de turbulences d'air dans l'habitacle, et des deuxièmes capteurs à ultrasons (US2) et/ou des deuxièmes microphones (M2) sont prévus pour détecter des bruits parasites, dans lequel l'unité d'évaluation et de commande (10) utilise les signaux bruts des deuxièmes capteurs à ultrasons (US2) et/ou des deuxièmes microphones (M2) pour compenser des bruits parasites lors de l'évaluation des bruits de respiration.

3. Système de surveillance d'occupant (1) selon la revendication 1 ou 2, **caractérisé en ce que** l'unité d'évaluation et de commande (10) transmet après un accident l'état de santé physique des différents occupants avec un appel d'urgence à une centrale d'appels d'urgence.

4. Système de surveillance d'occupant (1) selon l'une quelconque des revendications 1 à 3, **caractérisé en ce que** l'unité d'évaluation et de commande (10) détermine la position et la posture des différents occupants par fusion et évaluation des signaux bruts.

5. Système de surveillance d'occupant (1) selon la revendication 1 ou 2 et 4, **caractérisé en ce que** pendant un déplacement automatisé, l'unité d'évaluation et de commande (10) décide sur la base de la posture et de l'état de santé physique d'un conducteur si le contrôle du véhicule sera à nouveau conféré au conducteur ou si le véhicule sera garé en sécurité de façon automatisée.

6. Système de surveillance d'occupant (1) selon la revendication 4 ou 5, **caractérisé en ce que** l'unité d'évaluation et de commande (10) calcule des positions des visages des différents occupants par localisation par écho.

7. Système de surveillance d'occupant (1) selon la revendication 1 à 6, **caractérisé en ce que** le système de capteurs d'habitacle (20) comprend au moins un capteur d'habitacle (IS1, IS2) supplémentaire dont les signaux sont utilisés par l'unité d'évaluation et de commande (10) pour la vérification de plausibilité et/ou l'optimisation du résultat d'évaluation des signaux bruts des capteurs à ultrasons (US1, US2) et/ou des microphones (M1, M2).

8. Système de surveillance d'occupant (1) selon la revendication 7, **caractérisé en ce que** ledit au moins un capteur d'habitacle (IS1, IS2) supplémentaire est réalisé sous forme de capteur de position assise ou de capteur optique ou de système lidar.

9. Système de protection des occupants, comprenant un système de surveillance d'occupant (1) selon l'une quelconque des revendications 4 à 8, dans lequel le système de protection des occupants décide pendant un accident sur la base de la position et de la posture des différents occupants des moyens de protection (SM1, SM2, SM3) qui seront activés pour la protection des occupants.
